# EUROPEAN PATENT APPLICATION

(11) **EP 3 360 954 A1**
(43) Date of publication of application: **15.08.2018**
(21) Application number: 17155144.3
(22) Date of filing: 08.02.2017
(51) Int. Cl.: C12M 1/00, C12M 1/09, C12M 1/12, C12M 1/26

(54) **FLOATING BIOFILM**

(71) Applicant: Wageningen Universiteit, 6708 PB Wageningen (NL)
(72) Inventor: JANSSEN, Marcel Gerard Jozef, 6717 TA EDE (NL); WIJFFELS, René Hubertus, 6703 AR WAGENINGEN (NL)
(74) Representative: EP&C

(57) **Abstract**

The present invention relates to a system and method for cultivating and/or growing microorganisms capable of photosynthesis. The system comprises at least one body of water containing essential nutrients for the microorganisms; at least one sheet which floats, or is held afloat, on the at least one body of water; at least one layer of microorganisms capable of photosynthesis covering at least part of the upper surface of the at least one sheet; and a gas preferably comprising carbon dioxide above the at least one sheet. The system can be used in a method for cultivating and/or growing microorganisms capable of photosynthesis.

## Description

### Technical field

The present invention relates to a system and method for cultivating and/or growing microorganisms capable of photosynthesis.

### Background of the invention

Growth and cultivation of microalgae (including all photosynthetic single celled eukaryotic algae, microphytes and cyanobacteria) have been employed for the production of food, feed, chemicals and energy carriers, as well as for the production of specific food and feed constituents. In addition, microalgae cultivation can be used to remove nutrients from wastewater and at the same time produce biomass for the above-mentioned applications, or for soil conditioning and fertilization.

Currently, the most used large-scale production system is based on open raceway ponds. In such systems only a limited number of specific species can be grown which are very competitive and will not be easily outgrown by other microorganisms. Biomass density reached in production ponds is low (less than 0.05 % w/v) necessitating capital and energy intensive dewatering steps.

The alternative, large-scale applications of closed photobioreactors, is still in its infancy and thus expensive. Currently many different pilot-scale systems are being tested for their reliability, scalability, and economic feasibility. WO2014064602 for example describes a photobioreactor comprising a sealed, covered plastic tubular sleeve coated with an aqueous layer of microalgae. However, this system grows the algae in suspension, i.e. within the aqueous layer, which requires dewatering when harvesting. Further, the suspension needs to be kept in motion to allow homogenous access to light for the whole culture. These aspects make that the system is energy and capital intensive. In addition, the productivity of the system leaves something to be desired, because the density of the biomass in the suspension is low.

Porous substrate bioreactors (PSBRs) have been proposed to solve some of the problems described above related to the high liquid volume and low culture densities. PSBRs grow microalgae immobilized in dense biofilm cultures. The drawback of these porous substrate bioreactors is that the supply of water and nutrients to the biofilm is particularly challenging, which is at the expense of biomass production, or results in reactor designs which are complex and not scalable.

The current systems thus have considerable disadvantages, including low productivity, high costs of algal harvesting (dewatering), and high costs of the structures required. Further, they often are not scalable to sizes required for large scale production. Specifically, the supply of water and sufficient nutrients to the microalgae is challenging.

It is an objective of the present invention to overcome at least one of the above disadvantages.

### Summary of the invention

The present inventors found that microalgal biofilms can be grown with high productivity and superior biomass density on floating woven or non-woven cloths. First trials were performed while growing *Chlorella sorokiniana* and *Acutodesmus obliguus.*

Optimal productivity can be easily reached because continuously and homogenously wetting the floating cloth is very simple as the floating cloth will always remain wet because it is in constant contact with water. Very large surfaces can be covered with such cloths and these non-wovens, or wovens, can be mass produced on very large rolls, i.e. more than 100 meters in length and a width of several meters. Consequently, this new production system greatly reduces the investment costs in large scale systems for microalgae production. At the same time, process conditions (nutrient supply, carbon dioxide supply, temperature, light source) can be controlled at close to optimal conditions. Specifically, the application of a large liquid body underneath the floating biofilm is advantageous as it acts as a heat sink during daytime preventing a too rapid increase of biofilm temperature. During night time, the same heat sink will provide heat thereby preventing a too dramatic decrease in temperature of the biofilm. Temperature control is one of the most important cost and energy factors in large-scale microalgae production.

### Detailed description of the invention

The present disclosure relates to a system which can be used for cultivating and/or growing microorganisms capable of photosynthesis, wherein the system comprises:
(i) at least one body of water containing essential nutrients for cultivating and/or growing the microorganisms;
(ii) at least one sheet which floats, or is held afloat, on the at least one body of water, the sheet having
   - a water absorption capacity of at least 30 ml water per 100 g sheet,
   - preferably a porosity of between 25-99%; and
   - a thickness of between 0.2 - 10 mm;
(iii) preferably at least one layer, or biofilm, of microorganisms capable of photosynthesis covering at least part of the upper surface of the at least one sheet, wherein preferably the microorganisms are allowed to receive photosynthetically active radiation;
(iv) a gas above the at least one sheet, the gas preferably having a carbon dioxide concentration of at least 0.1 vol.%

There is no specific limitation with respect to the microorganisms to be cultivated and/or grown with the system. Advantageously, the microorganisms are capable of forming a biofilm, i.e. they can form a layer in which cells stick to each other and adhere to a surface. The biofilm is preferably allowed to receive photosynthetically active radiation, for example by placing the system out of doors, i.e. in sunlight (during daytime), and/or artificial light (during day and/or night time). With photosynthetically active radiation, in the context of the present disclosure, is meant at least part of the spectral range (wave band) of (solar) radiation that photosynthetic organisms are able to use in the process of photosynthesis (converting light energy into chemical energy), especially the range from 400 to 750 nm.

Therefore, preferably , the microorganisms are capable of photosynthesis (photoautotrophic), and typically they are unicellular. For example they can be chosen from
- microalgae, preferably of the genera *Chlorella, Acutodesmus, Botryococcus, Neochloris, Ettlia, Monoraphidium, Stichococcus, Nannochloropsis, Phaedactylum, Rhodomonas, Porhyridium,Tetraselmis, Euglena, Isochrysis, Dunaliella, Chlamydomonas, Haematococcus, Chroomonas, Chromochloris, Emiliana, Pavlova, Chaetoceros, Nitzschia, Cyclotella,* or *Thalassiosira*; and/or
- cyanobacteria and other phototropic bacteria, preferably of the genera *Acaryochloris*, *Anabaena, Arthrospira, Chlorobium, Cyanothece*, *Gloeobacter, Microcystis, Nostoc, Prochlorococcus, Spirulina, Synechocystis, Synechococcus, Thermosynechococcus, Trichodesmium, Rhodopseudomonas, Rhodobacter.*

With cultivating and/or growing is meant that the system can enable to maintain the microorganisms alive and/or increase biomass thereof, e.g. by replication of microorganisms.

The characteristics of the at least one sheet makes it possible to load the sheet with any type of microorganism, in particular any type of microalgae. For example, by dipping or immersing the sheet in a suspension of microalgae it can be instantly inoculated. With the term "sheet" is meant something, i.e. a layer that is thin in comparison to its length and width. A sheet can thus cover a significant area with limited material. If applied according to the present disclosure, the high porosity of the sheet enables diffusion of nutrients from the at least one body of water to the microorganisms.

The water absorption capacity of the at least one sheet allows for fast and homogenous wetting thereof. Water absorption capacity can be measured for example by using the method of ISO 2417. A test piece of known mass is immersed in water for 5 minutes, and the volume of the water absorbed is measured. Although not limited thereto, the at least one sheet has a water absorption capacity of at least 20, 30, or at least 50 ml water per 100 g sheet, preferably at least 75, 100, 150, 200, 300, 400, 500 or even at least 600 ml water per 100 g sheet.

A high porosity of the at least one sheet allows for improved diffusion of nutrients from the at least one water body provided the empty space can be filled with water and not merely with gas such as air. Porosity can for example be measured in terms of the void (empty) spaces in a material, and is the fraction of the voids in relation to the total volume, as a percentage between 0% and nearly 100%, where a higher percentage indicates a higher porosity (more void volume). Industrial CT scanning can for example be used to determine porosity. Preferably, the at least one sheet has a porosity of between 25-99%, more preferably between 40-80%, or 50-80%, 60-99% or between 70-99%.

Additionally, it is preferred that (at least 80% of the area of) the at least one sheet has an average pore size of between 10-50 µm, or 10-20 µm, or 2-15 µm, or between 5-15 µm, or even smaller than 20, 15, 10, 5, 4, 3, 2 µm, which may help retain the microorganisms on the at least one sheet and/or prevent the microorganisms from penetrating the sheet or even pass through the sheet into the body of water. Alternatively, the at least one sheet has, or is carried (or held afloat) by, an underlying layer, sheet, or filter, having such characteristics.

The (average) thickness of the at least one sheet preferably is chosen between 0.2-10 mm, preferably between 0.3-5 mm, 0.3-3 mm, or between 0.5-2 mm, or between 0.5-1 mm, which can be measured with appropriate means with a wetted sheet (upon 5 min immersion in water, or when floating), for example by measuring three random places of the sheet and averaging the thickness. A thick layer of biofilm supporting sheet (cloth) is advantageous for increased buoyancy, which allows to carry more biomass. On the other hand, increased thickness will reduce the diffusion rates of nutrients from the underlying water body through the at least one sheet towards the biofilm.

Since it is preferred that the at least one sheet floats or is held afloat, its density may for example be chosen between 0.01 - 0.99 g/cm³, preferably between 0.01 - 0.95 g/cm³, more preferably between 0.1 - 0.95 g/cm³, or 0.2 - 0.95 g/cm³, or 0.3 - 0.90 g/cm³. Simultaneously, or alternatively, the at least one sheet may be held afloat by an underlying sheet or underlying objects. The buoyance of the sheet can thus for example be enhanced by an underlying sheet or mesh for example made of at least 80 wt.% polypropylene (PP) or polyethylene (PE). Alternatively buoyancy can be increased by applying a bed of floating granulates (e.g. spheres) of PP or PE . Alternatively any material with a density lower than water can be combined with the biofilm carrying sheet.

The at least one sheet can be a woven or non-woven fabric. A non-woven fabric may have the advantage of having smaller pores, which may help retain and/or prevent penetration of microorganisms through the sheet as mentioned herein before. Simultaneously, or alternatively, it may have a surface area of at least 1 m², preferably at least 10, or 50, 100, 200, 300, 400, or even 500 m². The sheet may for example have a length of 1-100 m, a width of 0.5-5 m, and a thickness as described elsewhere herein. As will be clear, there can be one, two, three or more separate layers of microorganisms covering one, two, three or more separate sheets which float on one two, three or more separate bodies of water.

There is no specific limitation for the material to be used for the at least one sheet. For example, the at least one sheet may comprise at least 20, 30, 40, 50, 60, 70, 80, 90 wt.% or 100 wt.% of
- polyurethane (PU);
- cellulose-viscose;
- polyester;
- polypropylene (PP); and/or
- polyethylene (PE).

Alternatively, or at the same time, the at least one sheet may comprise at least 20, 30, 40, 50, 60, 70, 80, 90 wt.% or 100 wt.% of
- Nylon;
- Modacrylic fiber;
- Olefin fiber;
- Acrylic fiber; and/or
- Polyester fiber.

Alternatively, or at the same time, the at least one sheet may comprise at least 20, 30, 40, 50, 60, 70, 80, 90 wt.% or 100 wt.% of
- Rayon artificial silk;
- Vinyon fibers;
- Saran®;
- Spandex fibers;
- Vinalon;
- Aramid fibers - known as Nomex, Kevlar and Twaron;
- Modal fibers;
- Dyneema®/Spectra; and/or
- PBI (Polybenzimidazole fiber).

The density of PE and PP is slightly lower than that of water and hence such layer will stay afloat in calm water bodies. Moreover, in the case of a non-woven material the capillary force will allow for an equal wetting of the layer allowing for a homogenous growth of the microalgae biofilm all across the surface.

It is particularly advantageous to use a sheet comprised of at least 50, 60, 70, 80, 90 wt.% or 100 wt.% of the combination (cellulose-)viscose and polypropylene, wherein the at least one sheet preferably is non-woven. For example, the sheet may have an inner layer (non-woven) comprising at least 80, or 90 wt.% cellulose-viscose and at least 1,2,3 wt.% polypropylene. On both sides, i.e. on top and on the bottom, a PU coating is applied, covering at least 80, or 90% of the surface. The cellulose-viscose binds water very well (-OH groups), which increases the hydrophilic characteristic of the sheet, and thereby its water absorption capacity to allow fast and homogeneous wetting. The PU coating may be applied on the upper side of the sheet only, or on the bottom side only, or may even be applied throughout the material. The PU coating is very smooth and may have many small pores, which ensures that the water remains absorbed.

In the above embodiment, or as separate characteristic, the at least one sheet may comprise, or may be coagulated with, a coating of polyurethane.

The application of a water body underneath the floating sheet supporting the biofilm allows for good temperature control. The thermal inertia of this water body volume allows for a large capacity of heat buffering during day time as well as night time, similar to that in traditional microalgae production ponds.

The body of water preferably has an average depth of between 5-200 cm, preferably between 10-100 cm. Additionally, or alternatively, the body of water:
- contains a source of nitrogen (for example nitrate, ammonia, or urea), and a source of phosphor (for example phosphate), which are the essential nutrients as referred to herein;
- has an average temperature of between 5-40, preferably between 20-35 degrees Celsius
- has a pH of between 5-10.5, preferably between 6-8 or between 8.5-10.5; and/or
- has a salinity of between 1.5-50, 1-350 or 1.5-20, or 40-60, preferably between 20-40 g/l.

The use of high strength wastewaters with more than 500 mg_{N}·L⁻¹ and more than 50 mg_{P}·L⁻¹ are ideally suited to support the biofilm growth. Or, said differently, the system accordingly to the present disclosure is ideally suited to treat high-strength nutrient-contaminated wastewaters.

The nutrient concentration sufficient for the microorganisms can be easily calculated or found through trial and error by the skilled person. Based on the thickness and porosity, the skilled person can calculate the rate of diffusion as a function of nutrient concentration in the liquid and compare these rates to the amount necessary to support biofilm growth. See Table 2. Further, one can work with a closed water body and monitor its composition and replenish only the nutrients taken up.

The nutrients required for microorganism growth are preferably provided by means of diffusion from the underlying water body, through the at least one sheet, towards the biofilm. The water body may be circulated through a separate water body with thermoregulation and pH control so as to maintain a constant temperature and constant pH.

In the situation where one wants to grow biomass for food or feed applications the high nutrient concentrations to be maintained may require the discharge of process waste with more than 50 mg_{N}·L⁻¹ of nitrogen and 9 mg_{P}·L⁻¹ of phosphorus. However, this can be circumvented to a high degree by recirculating the water. By regular monitoring of the nutrient concentration and replacing depleted nutrients this can be realized. Accumulation of salts can be prevented by carefully balancing different components by a procedure already described in Spaargaren, 1996, Journal of Biotechnology, 45(2), pp. 97-102.

As mentioned herein before, there is preferably a gas above the at least one sheet (and above the layer of microorganisms, i.e. the biofilm) according to the present disclosure. Exchange of oxygen and carbon dioxide takes place along this gas exposed side of the biofilm which is growing on the upper side of the at least one sheet. For this purpose the gas phase above the biofilm can be managed in order to reach above air-level carbon dioxide concentrations, for example so as to contain 0.2-20, 0.5-20, or 1-15, or 2-20, preferably 5-15 vol.% CO₂. Further, the gas may contain a source of nitrogen, for example in case the body of water does not contain (sufficient) source of nitrogen, e.g. with the aim to apply nitrogen limitation on the upper side of the biofilm.

The gas may at least partially be enclosed by a cover that is at least partially transparent to photosynthetically active radiation. In fact, the whole system may be covered by a transparent plastic film, transparent plastic plates, or transparent glass. On a large scale gas preferably flows over the biofilm in plug flow in order to maximize the concentration gradient of carbon dioxide and, as such, its absorption by the biofilm. At the same time loss of carbon dioxide via the outgoing gas stream can be minimized, for example as described by Blanken (2016, "Optimizing carbon dioxide utilization for microalgae biofilm cultivation", Biotechnology and Bioengineering, DOI: 10.1002/bit.26199).

However, it is also possible not to enrich the gas with CO₂. For example, dissolved inorganic carbon, CO₂ - HCO₃ - CO₃) can be added to the water body according to the present disclosure, preferably my means of bicarbonate (such as NaHCO₃ or KHCO₃). See for example Chi, Z., O'Fallon, J. V. & Chen, S., 2011, Trends in Biotechnology, 29(11), pp.537-541. A bicarbonate concentration of e.g. at least 0.1, 0.2, 0.3, 0.4, 0.5, 0.6, 0.7, 0.8, 0.9, 1.0 M can be used, and/or up to at most e.g. 0.5, 0.6, 0.7, 0.8, 0.9, 1.0, 1.1, 1.2, 1.3, 1.4, 1.5, 2.0 M, in the water body. It is also envisaged that the dissolved carbon is obtained from combustion gases.

Further, preferably, the upper surface of the at least one sheet carries less than 1, more preferably less than 0.5, 0.2 or 0.1 litre of water per 1 m² of the at least one sheet, which ensures efficient carbon dioxide diffusion. This amount of water can be measured by tilting the sheet and collecting the water that flows due to gravity.

The cover may have a curved shape or be belly shaped, in order to prevent formation of water droplets on the cover of the system dripping back on the biofilm which may disturb its structure and create thin water layers on top of the biofilm limiting gas exposure and thus limiting growth (decreased CO₂ supply and O₂ removal). In practice the cover thus could have a shape allowing the droplets to flow back along the surface back into the body of water which carries the sheet with the biofilm. For this reason, the system may have a transparent cover foil which is belly-shaped.

The present disclosure also provides for a method, preferably for cultivating and/or growing microorganisms capable of photosynthesis, wherein the method comprises
a) providing the system according to the present disclosure, which comprises the layer of microorganisms;
b) allowing the microorganisms to grow; and
c) collecting (e.g. harvesting) the microorganisms.

Preferably, step b) is performed for a period of between 1-30 days, or 4-15, 6-8 days in order to obtain considerable amounts of biomass. The optimal harvesting period depends on cultivation conditions but is in the above ranges depending on temperature and light levels, and depending on the microorganism species grown.

Step c) can be performed by scraping the microorganisms from the sheet. Harvesting can take place by scraping and/or squeezing the at least one sheet. This can be a manual action, or it can be easily automatized by rolling up the sheet and at the same time scrape and squeeze with a dedicated harvesting machine. As such, a productivity of 175 g·m⁻² biomass can be obtained. Remaining biomass attached on, and inside, the at least one sheet allows for rapid re-growth of the biofilm after harvesting.

Products that can be produced with the present system and method include:
- Live algal feed for aquaculture;
- Algal protein components for food and feed applications;
- Algal lipid components for food and feed applications, specifically omega-3 fatty acids (EPA and DHA);
- Algal lipid components as a feedstock for biofuels;
- Algal lipid components as a feedstock for chemistry (e.g. plastics);
- Algal pigments (antioxidants) for food and feed applications;
- Algal pigments for chemistry (e.g. colorants);
- Algal exopolysaccharides for food, feed, and cosmetics; and/or
- Any product that can be produced and secreted specifically by genetically modified microalgae or cyanobacteria or that can be harvested from these (e.g. short chain alcohols such as ethanol, propanol, and butanol, short-chain fatty acids such as acetate, hydrocarbons, polysaccharides, volatile components such as ethylene).

In some situations one may want to aim for nitrogen limitation at the sunlight exposed side of the biofilm, particularly in case one wants to induce accumulation of specific storage products by means of nitrogen limitation, or the limitation of any other nutrient. In this design, the sunlight reaches the biofilm from the side opposing the side from where the nutrients diffuse into the biofilm. As such, nutrient limitation can be tuned and targeted to the side of the biofilm with highest light exposure resulting in high product accumulation.

The present system and method can thus be used for growth and production of microalgae for the production of food, feed, chemicals and energy carriers, or for the production of specific food and feed constituents. In addition, it can be used to remove nutrients from wastewater and preferably at the same time produce biomass to be used as fertilizer and soil conditioner. Specifically high strength wastewaters (urine, digestate, centrate) can be treated efficiently since the high nutrient concentrations allow for rapid diffusion through the at least one sheet.

Methods of carrying out conventional techniques used in a system or method of the present disclosure will be evident to the skilled worker.

### Brief description of the figures

Figure 1: Schematic presentation of liquid phase + biofilm (positioning different layers as related to liquid and gas)
Figure 2: gas phase management 02 and CO2 exchange with floating biofilm

The following examples illustrate different embodiments of the invention.

### EXAMPLE

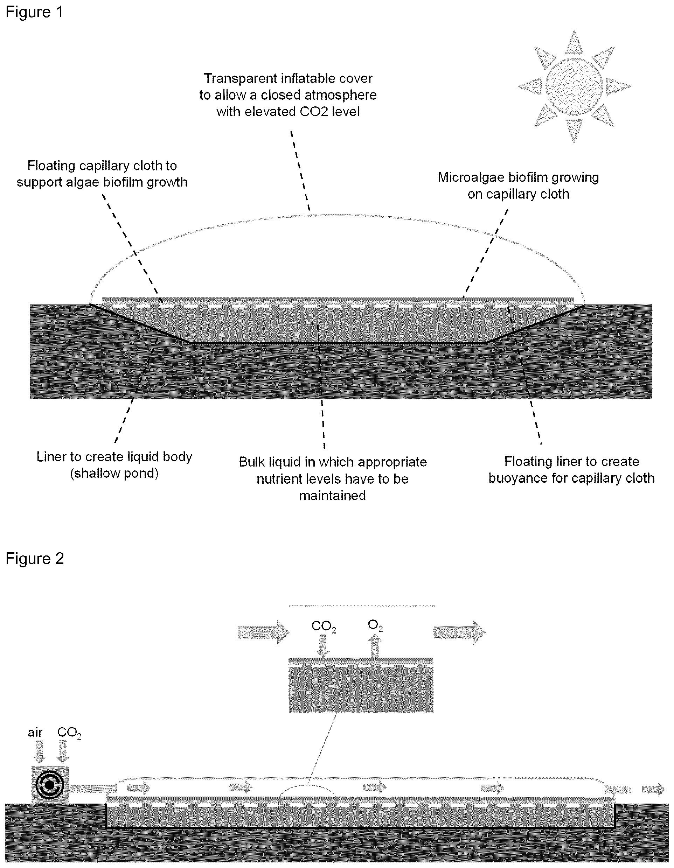

In this example, the floating biofilm system as according to the present invention is exemplified, see also Figure 1.

### Body of water

A container with a body of water was provided. The waterbody was recirculated through a water bath with thermoregulator to maintain a constant temperature (30°C) and at the same time the pH was measured and regulated between pH 6.5 and 7.0. The thermal inertia of the body of water allows for a large capacity of heat buffering during day time as well as night time, similar to that in traditional microalgae production ponds.

Standard freshwater microalgal cultivation media were used. Accordingly, the water body was enriched with nutrients based on a standard M-8a growth medium for *Chlorella* with nitrate as a nitrogen source (Kliphuis et al., 2010, Biotechnology progress 26(3), pp. 687-696). See Table 1 below.

**Table 1 Composition of M-8a medium.**

| Compound | Concentration (µmol L⁻¹) |
|---|---|
| KNO₃ | 29.67·10³ |
| KH₂PO₄ | 5.44 ·10³ |
| Na₂HPO₄ · 2 H₂O | 1.46 ·10³ |
| MgSO₄ · 7 H₂O | 1.62 ·10³ |
| CaCl₂ · 2 H₂O | 88.43 |
| EDTA ferric sodium salt | 315.86 |
| Na₂EDTA · 2 H₂O | 100.00 |
| H₃BO₃ | 1.00 |
| MnCl₂ · 4 H₂O | 65.59 |
| ZnSO₄ · 7 H₂O | 11.13 |
| CuSO₄ · 5 H2O | 7.33 |

(reflects the concentration in the water body. The nitrate salt KNO₃ can be replaced with ammonium salt.)

One can work with a closed water body and monitor its composition and replenish only the nutrients taken up. Accumulation of salts can be prevented by carefully balancing different components by a procedure described in a study by Spaargaren (1996, Journal of Biotechnology, 45(2), pp. 97-102).

For *Acetodesmus* the medium was adopted as described by Breuer (Breuer et al., 2013, Bioresource Technology, 143, pp.1-9) with increased nitrate concentration. Its composition is given by: KNO₃ 20mM; Na₂SO₄ 0.7 mM; MgSO₄.7H₂O 1mM; CaCl₂.2H₂O 0.5 mM; K₂HPO₄ 2.5 mM; NaHCO₃ 10mM; NaFeEDTA 28 µM; Na₂EDTA.2H₂O 80µM; MnCl₂.4H₂O 19µM; ZnSO₄.7H₂O 4µM; CoCl₂.6H₂O 1.2 µM; CuSO₄.5H₂O 1.3 µM; Na₂MoO₄.2H₂O 0.1 µM; Biotin 0.1 µM; vitamin B1 3.7 µM; vitamin B12 0.1µM.

### Sheet

As a capillary cloth a floating shammy was used, Chamlon P5050 (Chamlon B.V., Ootmarsum, The Netherlands). The underlying mesh was a PP mesh (PP65M, TopZeven B.V., Haarlem, The Netherlands).

The actual material was Chamlon P5050, a thin and flexible non-woven based on cellulose-viscose and polypropylene (PP) fibers and coagulated (coated) with polyurethane (PU).

This non-woven material appeared to have a considerable capillary force and a density lower than water making it float on water.

The cloth has a thickness of 0.80 mm, a dry weight of 178 g/m² and a wet weight of 781 g/m². This results in a relative water content of 0.74. These characteristics determine the diffusion rate of nutrients from the water body side of the cloth to the opposing biofilm side. When aiming at maximal diffusion driven nutrient transport through the cloth the layer thickness should be minimized. This, however, should be balanced with the biofilm carrying capacity (buoyancy) of a thicker cloth. Below an example calculation.

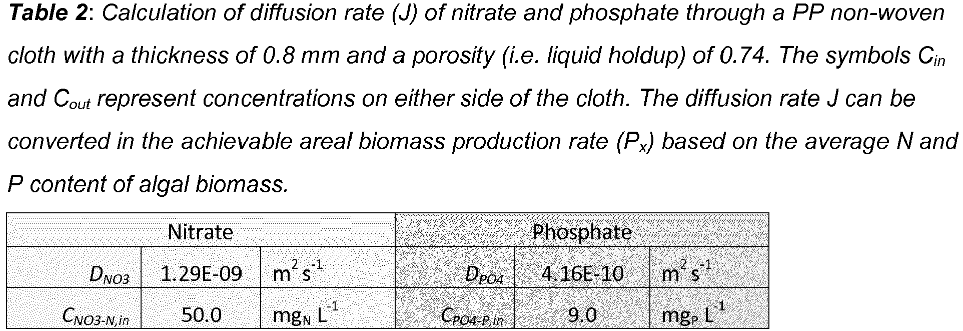

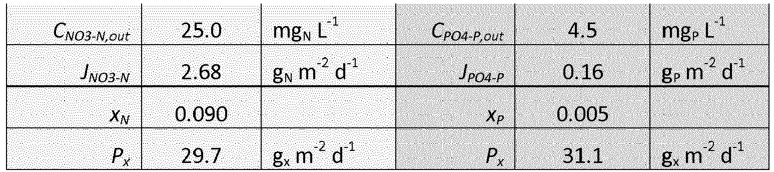

To creating surplus buoyancy an additional PP mesh (PP65M, TopZeven B.V., The Netherlands) was applied. This material has a weight of 216 g/m² and creates an additional upward force of 0.114 N·m⁻² creating sufficient buoyancy to keep the biofilm afloat.

### Layer of microorganisms capable of photosynthesis

The microalgal biofilm was grown on cloth as described above. Potentially any microalgae or cyanobacterium can be grown in a biofilm. Trials were carried out with strains of the species *Chlorella sorokiniana* and *Acutodesmus obliguus*, which were allowed to receive photosynthetically active radiation supplied by compact fluorescent lamps placed above the biofilm. Light intensity was set between 300 and 400 µmol/m² of PAR photons.

The capillary action of the cloth makes it possible to load the cloth with any specific microalgae species. More specifically by dipping the cloth in a microalgae suspension it can be instantly inoculated. In tests with the bench-scale system, an *Acetodesmus obliguus* biofilm and a *Chlorella sorokiniana* biofilm was grown. With *Chlorella* a productivity of between 12 to 18 g/m² was obtained for a consecutive period of 8 weeks.

### Gas phase

The gas phase above the biofilm must be managed in order to reach above air-level carbon dioxide concentrations (see Figure 2). In this way, exchange of oxygen and carbon dioxide takes place along the gas exposed side of the biofilm which is growing on the upper side of the floating cloth.

This is done by covering the whole system by a transparent plastic film, transparent plastic plates, or transparent glass. This strategy was also applied in the bench scale test where the gas phase was enriched to 10 vol.% CO₂. On a large scale, gas must preferably flow over the biofilm in plug flow in order to maximize the concentration gradient of carbon dioxide and, as such, its absorption by the biofilm. At the same time loss of carbon dioxide via the outgoing gas stream is minimized (see Blanken et al., 2016, Biotechnology and Bioengineering, DOI: 10.1002/bit.26199).

In practice the cover preferably has a shape allowing the droplets to flow back along the surface back into the liquid carrying the cloth with the biofilm, e.g. a curved or belly shape.

### Growing & harvesting

Optimal harvesting period depends on cultivation conditions but is in the range of 1 week to several weeks depending on temperature and light levels, and depending on the microalgal species grown.

Harvesting can take place by both scraping and squeezing the cloth. This can be easily automatized to create a continuous production and harvesting system: rolling up the cloth and at the same time scrape and squeeze with a dedicated harvesting machine. As such biomass densities between 10% and 25% w/v are achieved, depending on the ratio between a scraping and squeezing action. Remaining biomass attached on, and inside, the cloth allows for rapid re-growth of the biofilm after harvesting.

### Results

The biofilm system will result in a biomass production of between 12 to 18, or even up to 25 g·m⁻²·d⁻¹ (dry weight basis). When taking a 7 day production-harvest interval it will thus result in an areal biomass density of between 84 and 126, or even up to 175 g·m⁻² after 7 days (achieved for *Chlorella sorokiniana).*

## Claims

1. System for cultivating and/or growing microorganisms capable of photosynthesis, the system comprising:
(i) at least one body of water containing essential nutrients for cultivating and/or growing the microorganisms;
(ii) at least one sheet which floats, or is held afloat, on the at least one body of water, the sheet having
- a water absorption capacity of at least 30 ml water per 100 g sheet, and
- a thickness of between 0.2 - 5 mm;
(iii) at least one layer of microorganisms capable of photosynthesis covering at least part of the upper surface of the at least one sheet;
(iv) a gas above the at least one sheet, the gas preferably having a carbon dioxide concentration of at least 0.1 vol.%

2. System according to claim 1, wherein the microorganisms are
- microalgae, preferably of the genera *Chlorella, Acutodesmus, Botryococcus, Neochloris, Ettlia, Monoraphidium, Stichococcus, Nannochloropsis, Phaedactylum, Rhodomonas, Porhyridium,Tetraselmis, Euglena, Isochrysis, Dunaliella, Chlamydomonas, Haematococcus, Chroomonas, Chromochloris, Emiliana, Pavlova, Chaetoceros, Nitzschia, Cyclotella,* or *Thalassiosira*; and/or
- cyanobacteria or other phototropic bacteria, preferably of the genera *Acaryochloris, Anabaena, Arthrospira, Chlorobium, Cyanothece, Gloeobacter, Microcystis, Nostoc, Prochlorococcus, Spirulina, Synechocystis, Synechococcus, Thermosynechococcus, Trichodesmium, Rhodopseudomonas,* and/or *Rhodobacter,*.

3. System according to any one of the previous claims, wherein the at least one sheet
- has a water absorption capacity of at least 100 ml water per 100 g sheet;
- has a porosity of between 25-99%, preferably 70-90%; and/or
- has an average pore size of between 2 - 15 µm.

4. System according to any one of the previous claims, wherein the at least one sheet
- is a woven or non-woven fabric;
- has a density of between 0.01 - 0.95 g/cm³;
- has a thickness of between 0.5 - 1 mm; and/or
- has a surface area of at least 1 m²

5. System according to any one of the previous claims, wherein the at least one sheet comprises at least 75 wt.% of
- polyurethane;
- cellulose-viscose;
- polyester;
- polypropylene; and/or
- polyethylene.

6. System according to any one of the previous claims, wherein the at least one sheet comprises a coating of polyurethane.

7. System according to any one of the previous claims, wherein the at least one sheet is held afloat by an underlying sheet or underlying objects.

8. System according to any one of the previous claims, wherein the body of water has an average depth of between 5-200 cm, preferably between 10-100 cm.

9. System according to any one of the previous claims, wherein the body of water
- contains a source of nitrogen (N), and a source of phosphor (P);
- has an average temperature of between 5-40 degrees Celsius
- has a pH of between 5-10.5; and/or
- has a salinity of between 1.5-50 g/l.

10. System according to any one of the previous claims, wherein the upper surface of the at least one sheet carries less than 1 litre of water per 1 m² of the at least one sheet.

11. System according to any one of the previous claims, wherein the gas is at least partially enclosed by a cover that is at least partially transparent to photosynthetically active radiation.

12. System according to claim 11, wherein the cover has a curved shape or is belly shaped.

13. Method for cultivating and/or growing microorganisms capable of photosynthesis, the method comprising:
a) providing the system according to any one of claims 1-12;
b) allowing the microorganisms to grow;
c) collecting the microorganisms.

14. Method according to claim 13, wherein step b) is performed for a period of between 1-30 days.

15. Method according to any one of claim 13-14, wherein step c) is performed by scraping the microorganisms from the sheet.
